# EUROPEAN PATENT APPLICATION

(11) **EP 3 865 490 A1**
(43) Date of publication of application: **18.08.2021**
(21) Application number: 19870532.9
(22) Date of filing: 29.09.2019
(51) Int. Cl.: C07F 5/02, A61K 31/69, A61P 31/04

(54) **SIMPLE PREPARATION METHOD FOR VABORBACTAM**

(30) Priority: 12.10.2018 CN 201811191076
(71) Applicant: Xin Fa Pharmaceutical Co., Ltd, Dongying, Shandong 257500 (CN)
(72) Inventor: WANG, Baolin, Shandong 257500 (CN); QI, Yuxin, Shandong 257500 (CN); XU, Xin, Shandong 257500 (CN); LIU, Yuesheng, Shandong 257500 (CN)
(74) Representative: Proi World Intellectual Property GmbH
(86) International application number: PCT/CN2019/108981
(87) International publication number: WO 2020/073850

(57) **Abstract**

What is provided in the present invention is a simple method for preparing Vaborbactam, in which S-3-hydroxy-6-oxohexanoic acid ester is used as the starting material to finally form the Vaborbactam (I) by carrying out the procedures of hydroxyl protection, imidization, asymmetric addition from borane or borate compounds, amino deprotection, amidation, cyclization and hydrolysis. The present invention is suitable for commercial production by virtue of the advantages: widely available and low cost raw materials; safe, simple and convenient process steps; no rigorous reaction conditions; and environment-friendly reaction courses.

## Description

### Technical Field of the Invention

The present invention relates to a simple method for preparing Vaborbactam, and belongs to the field of pharmaceutical and biochemical engineering.

### Background of the Invention

Vaborbactam (I) is a novel cyclic boronic acid pharmacophore-based, non-β-lactam β-lactamase inhibitor, falling into Type A and C broad-spectrum β-lactamase inhibitors. This drug is discovered by Rempex Pharmaceuticals, Inc. and developed by The Medicines Company and the U.S. Department of Health and Human Services (HHS).

In August 2017, the U.S. Food and Drug Administration (FDA) approved the combination drug of Vaborbactam and Meropenem (Vabomere) for adults with complicated urinary tract infections (cUTI), including the pyelonephritis caused by sensitive enterobacteriaceae.

The chemical name for Vaborbactam (I), CAS No. 1360457-46-0, is (3R,6S)-{2-hydroxy-3-[2-(2-thiophene)acetamido]-1,2-oxaborinan-6-yl}acetic acid, and its chemical structural formula is shown as follows:

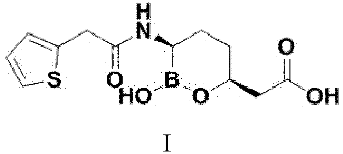

As described in the Journal of Medicinal Chemistry, 2015, 58, 3682-3692 and the patent document CN103180328A*,* R-3-hydroxy-4-pentenoic acid tert-butyl ester is used as the starting material for preparing Vaborbactam (I). R-3-hydroxy-4-pentenoic acid tert-butyl ester first reacts with tert-butyldimethylchlorosilane in the presence of imidazole to form hydroxyl protected pentenoic acid tert-butyl ester, and then reacts with pinacolborane under the catalysis of iridium to form Intermediate 4 with additive borohydride at the terminal. The Intermediate 4 reacts with (1S,2S,3R,5S)-(+)-2,3-pinanediol to form the Intermediate 5, which then reacts with methylene dichloride under the action of n-butyllithium at -95°C to form the Intermediate 6 with chiral carbon chain. The Intermediate 6 reacts with lithium bis(trimethylsilyl)amide drops, added at -78°C, for 16h at room temperature, and then condenses with 2-thiopheneacetic acid under the action of carboxylic activators EDCI and HOBT at 0°C to form the Intermediate 7. The Intermediate 7 is then cyclized and hydrolyzed in the presence of 3N hydrochloric acid to form the target product (I). See Reaction Pathway 1 for more information.

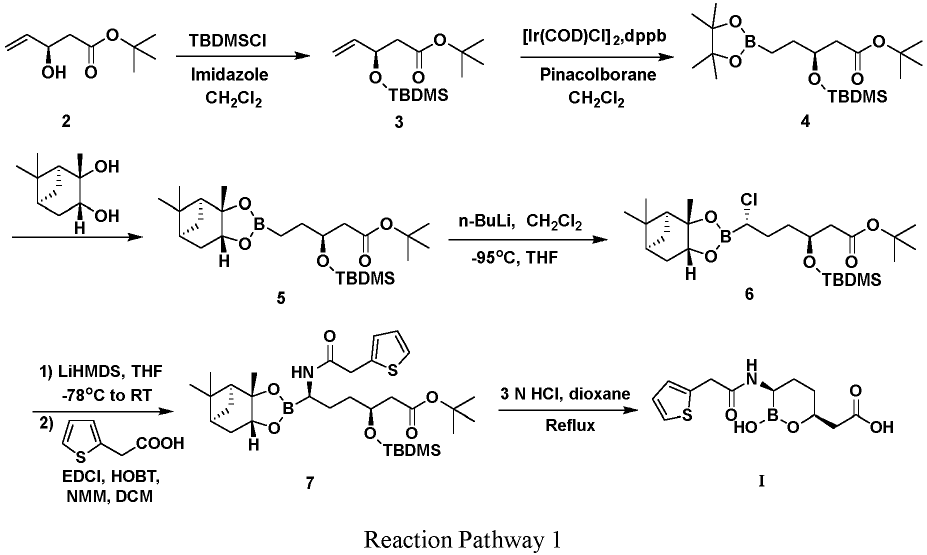

However, the raw materials used in the above Reaction Pathway 1 are not easy to obtain and expensive, and the reaction conditions are rigorous, including two steps to be carried out at extremely low temperature in addition to the use of lithium reagent, resulting in low operation safety level and other adverse effects on commercial production.

A synthesis method similar to the above Reaction Pathway 1 is disclosed in the patent document US20170057979, the difference is that in this method, bis(pinacolato)diboron is first hydrolyzed, and aminoethanol is used to generate a spiro compound, which then reacts with (1S,2S,3R,5S)-(+)-2,3-pinanediol. The subsequent reactions are the same, and finally IN dilute sulfuric acid and boric acid are used for cyclization and hydrolysis to obtain the target product I. See Reaction Pathway 2 for details.

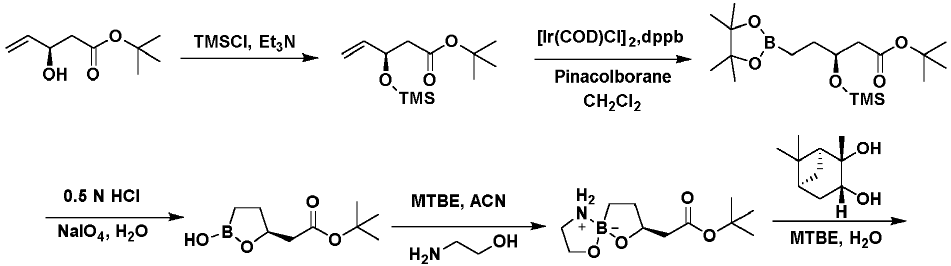

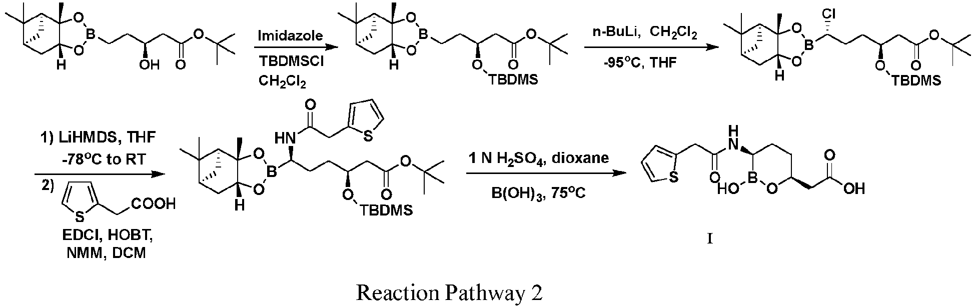

Similarly, the raw materials used in the above Reaction Pathway 2 are not easy to obtain and expensive, and the reaction conditions are rigorous, including two steps to be carried out at extremely low temperature in addition to the use of lithium reagent, resulting in low operation safety level and other adverse effects on commercial production.

### Summary of the Invention

To address the above-mentioned deficiencies of the prior art, the present invention is intended to provide a simple method for preparing Vaborbactam, namely (3R,6S)-{2-hydroxy-3-[2-(2-thiophene)acetamido]-1,2-oxaborinan-6-yl}acetic acid. The present invention is suitable for commercial production by virtue of the advantages: widely available and low cost raw materials; safe, simple and convenient process steps; no rigorous reaction conditions; and environment-friendly reaction courses.

### Terminology:

Compound of Structural Formula II: S-3-hydroxy-6-oxohexanoic acid ester;
Compound of Structural Formula III: S-3-(protecting group)oxy-6-oxohexanoic acid ester, wherein, PG refers to a Protecting Group;
Compound of Structural Formula IV: S-3-(protecting group)oxy-6-(N-(substituent)imi no)hexanoic acid ester;
Compound of Structural Formula V: (3S,6R)-3-(protecting group)oxy-6-(N-(substitue nt)imino)-6-borylhexanoic acid ester;
Compound of Structural Formula VI: (3S,6R)-3-(protecting group)oxy-6-amino-6-bor ylhexanoic acid ester; and
Compound of Structural Formula VII: (3S,6R)-3-(protecting group)oxy-6-boryl-6-[2-(2-thiophene)acetamido]hexanoic acid ester.

Each compound and corresponding structural formula as listed above are referred to by using the same serial number herein to indicate the same substance, while the structural formula is to be taken as the standard definition in case of any inconsistencies therebetween.

### Technical Solution:

A method for preparing Vaborbactam comprises the steps as follows:
(1) Reacting a compound of Formula II with a hydroxyl protecting group reagent to form a compound of Formula III; 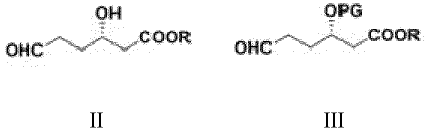
   In the Structural Formulas II and III, R is methyl, ethyl, isopropyl, n-propyl, n-butyl, isobutyl, sec-butyl or tert-butyl; in the Structural Formula III, the protecting group (PG) is trimethylsilyl (TMS), tert-butyldimethylsilyl (TBDMS), benzyl (Bn), methanesulfonyl (Ms), p-toluenesulfonyl (Ts), trifluoroacetyl (TFA) or acetyl (Ac);
(2) Reacting a compound of Formula III with an amine compound by imidization to form a compound of Formula IV; 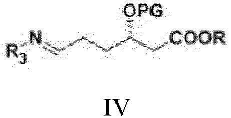
   In the Structural Formula IV, R₃ is hydroxyl, benzoyl, phenylacetyl, 2-thiopheneacetyl or alkylsulfinyl, and the meanings of R and PG are the same as those in Formula III;
(3) Reacting a compound of Formula IV with a borane or borate compound to form a compound of Formula V; 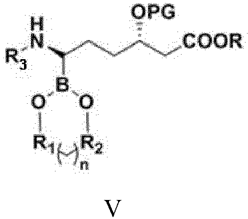
   In the Structural Formula V, n is 0, 1, 2 or 3; when n is 0, R₁ and R₂ are alkyl or aryl, and preferably, R₁ and R₂ are phenyl, methylphenyl or chlorophenyl, or alkyl having 1 to 4 carbon atoms; when n is 1, 2 or 3, R₁ and R₂ are alkyl having 1 to 4 carbon atoms, and preferably, R₁ and R₂ are ethylene (-CH₂CH₂-) or substituent ethylene; R₁ and R₂ are the same or different; and the meanings of R, PG, and R₃ are the same in Formula IV;
(4) Deprotecting a compound of Formula V to remove the protecting amino to form a compound of Formula VI; 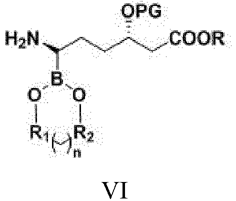
   In the Structural Formula VI, the meanings of R₁, R₂, n, R and PG are the same as those in Formula V;
(5) Amidating a compound of Formula VI in the presence of an amidation reagent to form a compound of Formula VII; 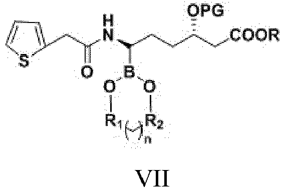
   In the Structural Formula VII, the meanings of R₁, R₂, n, R and PG are the same as those in Formula V;
(6) Cyclizing and hydrolyzing a compound of Formula VII to form Vaborbactam (I).

As disclosed in the present invention, in Step (1), the compound of Formula II can be acquired on the market or prepared with the available technologies.

For the purpose of the present invention, it is preferable to carry out Step (1) by reacting the compound of Formula II with a hydroxyl protecting group reagent in a Solvent A and in the presence of a base.

Preferably, the Solvent A is a non-alcoholic solvent, more preferably ethyl acetate, butyl acetate, acetone, methyl isobutyl ketone, acetonitrile, tetrahydrofuran, 2-methyltetrahydrofuran, 2,4-dioxane, methoxycyclopentane, methyl tert-butyl ether, or halogenated hydrocarbon solvents or benzene solvents, or any combination thereof; and the mass ratio of Solvent A to the compound of Formula II is (4 ∼ 20):1, preferably (5 ∼ 10):1.

Preferably, the base is an organic base or inorganic base, and more preferably, the organic base is trimethylamine, triethylamine, tri-n-butylamine, diisopropylethylamine or imidazole, while the inorganic base is potassium carbonate, sodium carbonate or calcium carbonate, or any combination thereof; and the molar ratio of the base to the compound of Formula II is (1.0 ∼ 2.0): 1, more preferably (1.1 ∼ 1.5):1.

Preferably, the hydroxyl protecting group reagent is trimethylchlorosilane, trimethyliodosilane, tert-butyldimethylchlorosilane, tert-butyldimethyliodosilane, methanesulfonyl chloride, p-toluenesulfonyl chloride , benzyl chloride, benzyl bromide, trifluoroacetic acid or acetic anhydride; and the molar ratio of the hydroxyl protecting group reagent to the compound of Formula II is (1.0 ∼ 2.0): 1, more preferably (1.1 ∼ 1.5): 1.

Preferably, the reaction temperature of the compound of Formula II and the hydroxyl protecting group reagent is 0-60°C, more preferably, 20 ∼ 40°C; and the reaction time of the compound of Formula II and the hydroxyl protecting group reagent is 1 ∼ 7 hours, more preferably 2 ∼ 5 hours.

For the purpose of the present invention, it is preferable to carry out Step (2) by imidizing the compound of Formula III by using an amine compound in a Solvent B under the action of a Catalyst C.

Preferably, the Solvent B is methanol, ethanol, isopropanol, acetonitrile, tetrahydrofuran, 2-methyltetrahydrofuran, 2,4-dioxane, methoxycyclopentane, methyl tert-butyl ether, or halogenated hydrocarbon solvents or benzene solvents, or any combination thereof; and the mass ratio of Solvent B to the compound of Formula III is (1 ∼ 20): 1, more preferably (2 ∼ 10):1.

Preferably, the Catalyst C is acetic acid, methanesulfonic acid, benzenesulfonic acid, p-toluenesulfonic acid, pyridine p-toluenesulfonic acid, copper sulfate, copper acetate, copper chloride, cuprous chloride or ferric chloride, or any combination thereof; and the molar ratio of Catalyst C to the compound of Formula III is (1 ∼ 3):1.

Preferably, the amine compound is hydroxylamine, benzamide, phenylacetamide, thiopheneacetamide or alkylsulfenamide; and the molar ratio of the amine compound to the compound of Formula III is (1.0 ∼ 2.0): 1.

Preferably, the imidization reaction is carried out at a temperature of -10 ∼ 100°C, more preferably 0 ∼ 70°C, and most preferably 30 ∼ 50°C.

For the purpose of the present invention, it is preferable to carry out Step (3) by reacting the compound of Formula IV with a borane or borate compound in a Solvent D in the presence of a Catalyst E and a ligand.

Preferably, the Solvent D is methanol, ethanol, isopropanol, acetonitrile, tetrahydrofuran, 2-methyltetrahydrofuran, 1,3-dioxane, methoxycyclopentane, methyl tert-butyl ether, water, or halogenated hydrocarbon solvents or benzene solvents, or any combination thereof; and the mass ratio of Solvent D to the compound of Formula IV is (2 ∼ 20):1, more preferably (2 ∼ 10):1.

Preferably, the Catalyst E is copper sulfate, copper chloride, cuprous chloride, palladium chloride, palladium acetate, tris(triphenylphosphine)rhodium(I) chloride, Grubb's catalyst, iridium/alumina or (1, 5-cyclooctadiene)(pyrimidine)(tricyclohexylphosphine)iridium(I) hexafluorophosphate; and the molar quantity of Catalyst E is 1.0 ∼ 10.0% of that of the compound of Formula IV, more preferably 1.0 ∼ 5.0%.

Preferably, the ligand is nitrogen ligand or phosphorus ligand, or more preferably a combination thereof; more preferably, the nitrogen ligand is substituted imidazole or benzylamine, and the phosphorus ligand is triphenylphosphine or triphenylphosphine oxide; and the molar quantity of ligand is 1.0 ∼ 10.0% of that of the compound of Formula IV, more preferably 1.0 ∼ 7.0%.

Preferably, the borane is dialkylborane or pinacolborane; the borate compound is trialkyl borate or biborate; the molar ratio of the borane or borate compound to the compound of Formula IV compound is 1.0 ∼ 2.0:1.

Preferably, the reaction of the compound of Formula IV with the borane or borate compound is carried out at a temperature of 20 ∼ 120°C, more preferably 20 ∼ 40°C.

For the purpose of the present invention, it is preferable to carry out Step (4) by deprotecting the compound of Formula V in a Solvent F and in the presence of a deprotection reagent.

Preferably, the Solvent F is methanol, ethanol, isopropanol, acetonitrile, tetrahydrofuran, 2-methyltetrahydrofuran, 2,4-dioxane, methoxycyclopentane, methyl tert-butyl ether, water, or halogenated hydrocarbon solvents or benzene solvents, or any combination thereof; and the mass ratio of the Solvent F to the compound of Formula V is 1 ∼ 20:1.

Preferably, the deprotection reagent is an acid or a base; the acid is hydrogen chloride, sulfuric acid or phosphoric acid; the hydrogen ion concentration in the acid is 3 ∼ 8mol/L; the base is sodium hydroxide, potassium hydroxide, barium hydroxide, sodium carbonate or potassium carbonate; the molar ratio of the deprotection reagent to the compound of Formula V is 3 ∼ 7:1.

Preferably, the deprotection is carried out at a temperature of -10 ∼ 100°C, more preferably -5 ∼ 30°C.

For the purpose of the present invention, it is preferable to carry out Step (5) by amidating the compound of Formula VI with the amidation reagent in a Solvent G under the action of the Base H.

Preferably, the Solvent G is methanol, ethanol, isopropanol, acetonitrile, tetrahydrofuran, 2-methyltetrahydrofuran, 2,4-dioxane, 1,4-dioxane, methoxycyclopentane, methyl tert-butyl ether, or halogenated hydrocarbon solvents or benzene solvents, or any combination thereof; and the mass ratio of the Solvent G to the compound of Formula VI is 4 ∼ 20:1, more preferably 4 ∼ 10:1.

Preferably, the amidation reagent is 2-thiopheneacetyl chloride or 2-thiopheneacetic acid; and the molar ratio of the amidation reagent to the compound of Formula VI is 1 ∼ 2.0:1.

More preferably, when the amidation reagent is 2-thiopheneacetic acid, a dehydrating and condensing agent is required; the dehydrating and condensing agent is dicyclohexylcarbodiimide (DCC), 1-ethyl-3-(3-dimethylaminopropyl)carbodiimide hydrochloride (EDCI) or 1-hydroxybenzotriazole (HOBT), or any combination thereof; and the molar ratio of the dehydrating and condensing agent to the compound of Formula VI is 1.0 ∼ 3.0:1.

Preferably, the Base H is an organic base or inorganic base; the organic base is trimethylamine, triethylamine, tri-n-butylamine, diisopropylethylamine, imidazole, morpholine or N-methylmorpholine; the inorganic base is potassium carbonate, sodium carbonate or calcium carbonate, or any combination thereof; the molar ratio of the Base H to the compound of Formula VI is (1.0∼3.0):1.

Preferably, the amidation is carried out at a temperature of 0 ∼ 120°C, more preferably 15 ∼ 80°C.

For the purpose of the present invention, it is preferable to carry out Step (6) by cyclizing and hydrolyzing the compound of Formula VII in a Solvent J and in the presence of an acid.

Preferably, the Solvent J is methanol, ethanol, isopropanol, acetonitrile, tetrahydrofuran, 2-methyltetrahydrofuran, 1,4-dioxane, methoxycyclopentane, methyl tert-butyl ether, or halogenated hydrocarbon solvents or benzene solvents, or any combination thereof; and the mass ratio of the Solvent J to the compound of Formula VII is 4 ∼ 20:1, more preferably 2 ∼ 10:1.

Preferably, the acid is hydrochloric acid, sulfuric acid, boric acid or trifluoroacetic acid, or any combination thereof; and the molar ratio of the acid to the compound of Formula VII is 5 ∼ 7:1.

Preferably, the cyclization and hydrolysis are carried out at a temperature of 10 ∼ 100°C, more preferably 70 ∼ 95°C; and the time of cyclization and hydrolysis is 1 ∼ 10 hours.

The reaction described in the present invention is shown in the Reaction Pathway 3 as follows:

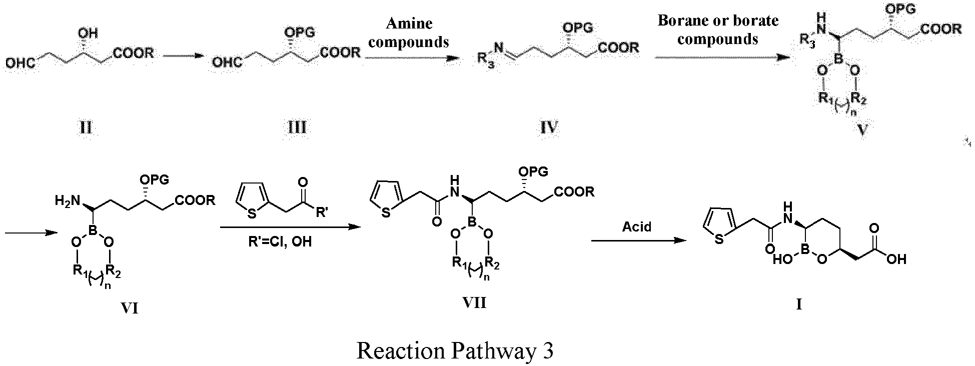

Wherein, R is methyl, ethyl, isopropyl, n-propyl, n-butyl, isobutyl, sec-butyl or tert-butyl; PG is trimethylsilyl (TMS), tert-butyldimethylsilyl (TBDMS), benzyl (Bn), methanesulfonyl (Ms), p-toluenesulfonyl (Ts), trifluoroacetyl (TFA) or acetyl (Ac); n is 0, 1, 2 or 3; when n is 0, R₁ and R₂ are alkyl or aryl; when n is 1, 2 or 3, R₁ and R₂ are alkyl having 1 to 4 carbon atoms; R₁ and R₂ are the same or different ; and R₃ is hydroxyl, benzoyl, phenylacetyl, 2-thiopheneacetyl or alkylsulfinyl.

### Technical Characteristics and Advantageous Effects of the Present Invention

The raw materials used for the present invention are cheap and easy to obtain, and can replace R-3-hydroxy-4-pentenoic acid tert-butyl ester or similar expensive materials, so that the cost can be reduced. The reactions involved in the present invention are classic, and the reaction conditions are easy to control and realize, to avoid the rigorous low-temperature reaction conditions, so that the energy consumption can be decreased. The dispensing with lithium reagents by the present invention may improve the safety level and simplicity of operation, as well as ensure the environment compliance, which is conducive to the green commercial production of Vaborbactam.

The reactions in each step of the present invention are classic and can ensure high yield under the flexible conditions, and the purity and yield of the reaction product in each step are relatively high, which is conducive to subsequent commercial production.

### Detailed Description of the Present Invention

The present invention is illustrated by but not limited to the following examples.

The raw materials and reagents used in the examples are all commercially available products, and the optical purity of S-3-hydroxy-6-oxohexanoic acid ester (II) is 99.6%.

Unless otherwise specified, the symbol "%" in the examples indicates a mass percentage, and the term "yield" indicates a molar yield.

A gas or liquid chromatograph is used to monitor the reaction process and product purity, and a liquid chromatograph equipped with a chiral column (ES-OVS, 150mm×4.6mm, Agilent) to detect the optical purity (area ratio, %), and calculate the yield as well as the value of ee.

### Example 1: Preparation of S-3-trimethylsilyloxy-6-oxo-hexanoic acid tert-butyl ester (III₁)

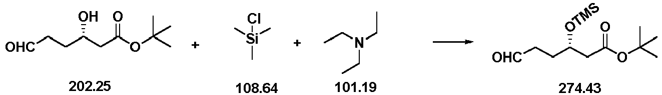

Under the protection of nitrogen, add 120g of dichloromethane, 20.2g (0.1mol) of S-3-hydroxy-6-oxo-hexanoic acid tert-butyl ester (II₁) and 12.1g (0.12mol) of triethylamine into a 250ml 4-necked flask equipped with a stirrer and a thermometer. Add a solution of 13.1g (0.12mol) of trimethylchlorosilane and 20g of dichloromethane dropwise and evenly across a time span of 30 minutes when the internal temperature is 15 ∼ 20°C. Then, stir to accelerate the reaction at 20 ∼ 25°C for 3 hours. Introduce the solution into 50g of water, and wait for separation. Extract the water layer twice with dichloromethane, 30g each time. Combine the organic phases, and wash twice with a saturated sodium chloride solution, 20g each time. Recover the solvents from the organic phases, to obtain 26.0g of S-3-trimethylsilyloxy-6-oxo-hexanoic acid tert-butyl ester (III₁) which has an LC purity of 99.9% and a yield of 94.9%.

### Example 2: Preparation of S-3-tert-butyldimethylsiloxy-6-oxo-hexanoic acid tert-butyl ester (III₂)

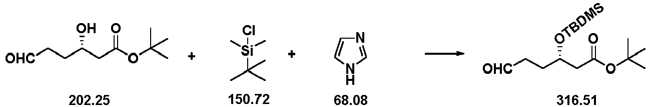

Under the protection of nitrogen, add 120g of dichloromethane, 20.2g (0.1mol) of S-3-hydroxy-6-oxo-hexanoic acid tert-butyl ester (II₁) and 8.2g (0.12mol) of imidazole into a 250ml 4-necked flask equipped with a stirrer and a thermometer. Add a solution of 16.6g (0.11mol) of tert-butyldimethylchlorosilane and 30g of dichloromethane dropwise and evenly across a time span of 30 minutes when the internal temperature is 15 ∼ 20°C. Then, stir to accelerate the reaction at 20 ∼ 25°C for 4 hours. Introduce the solution into 50g of water, and wait for separation. Extract the water layer twice with dichloromethane, 30g each time. Combine the organic phases, and wash twice with a saturated sodium chloride solution, 20g each time. Recover the solvents from the organic phases, to obtain 30.4g of S-3-tert-butyldimethylsiloxy-6-oxo-hexanoic acid tert-butyl ester (III₂) which has an LC purity of 99.9% and a yield of 96.0%.

### Example 3: Preparation of S-3-trimethylsilyloxy-6-(N-(R-1-tert-butylsulfinyl)imin o)hexanoic acid tert-butyl ester (IV₁)

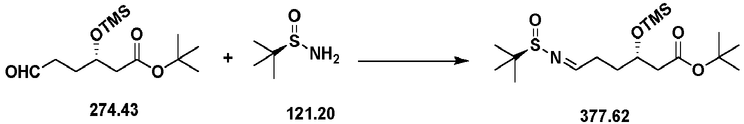

Under the protection of nitrogen, add 120g of dichloromethane, 27.4g (0.1mol) of S-3-trimethylsilyloxy-6-oxo-hexanoic acid tert-butyl ester (III₁) as obtained in Example 1, 2.5g (0.1mol) of pyridine p-toluenesulfonate, 31.9g (0.2mol) of copper sulfate and 13.3g (0.11mol) of R-tert-butylsulfonamide into a 250ml 4-necked flask equipped with a stirrer, a thermometer and a reflux apparatus. Reflux after heat to 40 ∼ 45°C, and stir at 40 ∼ 45°C till the reaction is confirmed as completed by using the HPLC. Filter to remove any insoluble matters. Reduce the pressure and distill to recover the dichloromethane. Separate and purify the residues by using the column chromatography (PE/EA=10:1), or rectify the residues, to obtain 36.0g of S-3-trimethylsilyloxy-6-(N-(R-1-tert-butylsulfinyl)imino)hexanoic acid tert-butyl ester (IV₁), which has an LC purity of 99.9% and a yield of 95.3%.

### Example 4: Preparation of S-3-dimethyl-tert-butylsilyloxy-6-(N-(R-1-tert-butylsul finyl)imino)hexanoic acid tert-butyl ester (IV₂)

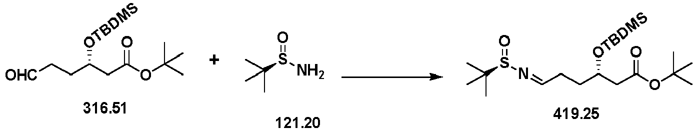

Under the protection of nitrogen, add 120g of dichloromethane, 31.7g (0.1mol) of S-3-tert-butyldimethylsiloxy-6-oxo-hexanoic acid tert-butyl ester (III₂) as obtained in Example 2, 2.5g (0.1mol) of pyridine p-toluenesulfonate, 31.9g (0.2mol) of copper sulfate and 13.3g (0.11mol) of R-tert-butylsulfonamide into a 250ml 4-necked flask equipped with a stirrer, a thermometer and a reflux apparatus. Reflux after heat to 40 ∼ 45°C, and stir at 40 ∼ 45°C till the reaction is confirmed as completed by using the HPLC. Filter to remove any insoluble matters. Reduce the pressure and distill to recover the dichloromethane. Separate and purify the residues by using the column chromatography (PE/EA=10:1), or rectify the residues, to obtain 36.0g of S-3-dimethyl-tert-butylsilyloxy-6-(N-(R-1-tert-butylsulfinyl)imino)hexanoic acid tert-butyl ester (IV₂), which has an LC purity of 99.9% and a yield of 95.3%.

### Example 5: Preparation of (3S,6R)-3-trimethylsilyloxy-6-(N-(R-1-tert-butylsulfiny l)amino)-6-(4,4,5,5-tetramethyl-1,3,2-dioxaborolan-2-yl)hexanoic acid tert-butyl ester (V₁)

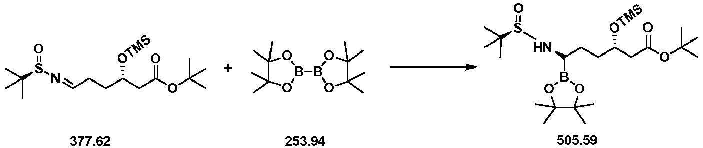

Add 100g of toluene, 20g of water, 0.2g (1.2mmol) of copper sulfate, 0.37g (1.2mmol) of triphenylphosphine oxide and 0.54g (5mmol) of benzylamine into a 250ml 4-necked flask equipped with a stirrer and a thermometer, and stir the solution. Add 37.7g (0.1mol) of S-3-trimethylsilyloxy-6-(N-(R-1-tert-butylsulfinyl)imino)hexanoic acid tert-butyl ester (IV₁) as prepared in Example 3 and 30.5g (0.12mol) of bis(pinacolato)diboron, and stir at the room temperature till the reaction is confirmed as completed by using the HPLC. Add 50g of ethyl acetate into the system. Filter, then reduce the pressure and distill to concentrate. Separate and purify the residues by using the column chromatography (EA/DCM=10:90), or rectify the residues, to obtain 39.2g of (3S,6R)-3-trimethylsilyloxy-6-(N-(R-1-tert-butylsulfinyl)amino)-6-(4,4,5,5-tetramethyl-1,3,2-dioxaborolan-2-yl)hexanoic acid tert-butyl ester (V₁), which has an LC purity of 98.6% and a yield of 77.6%.

### Example 6: Preparation of (3S,6R)-3-dimethyl-tert-butylsilyloxy-6-(N-(R-1-tert-b utylsulfinyl)amino)-6-(4,4,5,5-tetramethyl-1,3,2-dioxaborolan-2-yl)hexanoic acid tert-bu tyl ester (V₂)

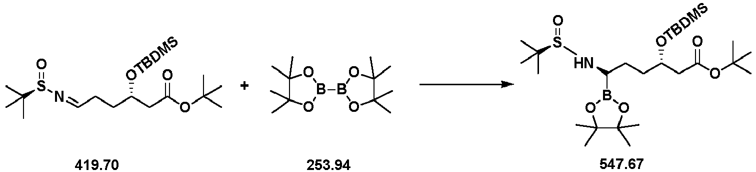

Add 100g of toluene, 20g of water, 0.2g (1.2mmol) of copper sulfate, 0.37g (1.2mmol) of triphenylphosphine oxide and 0.54g (5mmol) of benzylamine into a 250ml reaction flask equipped with a stirrer and a thermometer, and stir the solution. Add 42.0g (0.1mol) of S-3-dimethyl-tert-butylsilyloxy-6-(N-(R-1-tert-butylsulfinyl)imino)hexanoic acid tert-butyl ester (IV₂) as prepared in Example 4 and 30.5g (0.12mol) of bis(pinacolato)diboron, and stir at the room temperature till the reaction is confirmed as completed by using the HPLC. Add 50g of ethyl acetate into the system. Filter, then reduce the pressure and distill to concentrate. Separate and purify the residues by using the column chromatography (EA/DCM=10:90), or rectify the residues, to obtain 44.7g of (3S,6R)-3-dimethyl-tert-butylsilyloxy-6-(N-(R-1-tert-butylsulfinyl)amino)-6-(4,4,5,5-tetramet hyl-1,3,2-dioxaborolan-2-yl)hexanoic acid tert-butyl ester (V₂), which has an LC purity of 98.4% and a yield of 81.6%.

### Example 7: Preparation of (3S,6R)-3-dimethyl-tert-butylsilyloxy-6-amino-6-(4,4,5, 5-tetramethyl-1,3,2-dioxaborolan-2-yl)hexanoic acid tert-butyl ester hydrochloride (V I)

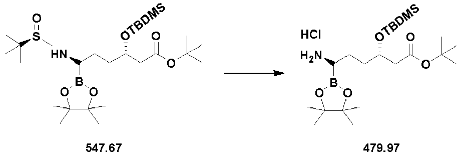

Add 100g of 20% methanol solution of hydrogen chloride to a 250ml reaction flask equipped with a stirrer and a thermometer, and cool to 0°C. Add 54.8g (0.1mol) of (3S,6R)-3-dimethyl-tert-butylsilyloxy-6-(N-(R-1-tert-butylsulfinyl)amino)-6-(4,4,5,5-tetramet hyl-1,3,2-dioxaborolan-2-yl)hexanoic acid tert-butyl ester (V₂) as prepared in Example 6, and then stir at 0°C till the reaction is confirmed as completed by using the HPLC. Reduce the pressure, distill and concentrate to obtain 47.3g of (3 S, 6R)-3 -dimethyl-tert-butylsilyloxy-6-amino-6-(4,4, 5,5-tetramethyl-1,3,2-dioxaborolan-2-yl )hexanoic acid tert-butyl ester hydrochloride, which has an LC purity of 99.2% and a yield of 98.6%.

### Example 8: Preparation of (3S,6R)-3-dimethyltert-butylsilyloxy-6-[2-(2-thiophene) acetamido]-6-(4,4,5,5-tetramethyl-1,3,2- dioxaborolan-2-yl)hexanoic acid tert-butyl ester (VII)

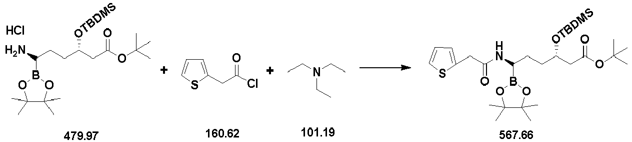

Add 100g of tetrahydrofuran, 24.0g (0.05mol) of (3 S, 6R)-3 -dimethyl-tert-butylsilyloxy-6-amino-6-(4,4, 5,5-tetramethyl-1,3,2-dioxaborolan-2-yl )hexanoic acid tert-butyl ester hydrochloride (VI) as prepared in Example 7, 9.6g (0.06mol) of 2-thiopheneacetyl chloride, and 11.1g (0.11mol) of triethylamine into a 250ml reaction flask equipped with a stirrer and a thermometer, and then stir at the room temperature till the reaction is confirmed as completed by using the HPLC. Add 80g of water and stir for 0.5 hours. Add 100g of ethyl acetate, and transfer to a separating funnel for static separation. Wash the organic phases with water twice, 30g each time. Combine the organic phases, and dry out with anhydrous sodium sulfate. Remove the sodium sulfate by filtering. Reduce the pressure of filtrate, and then distill to remove the solvents to form a pale yellow crude product. Recrystallize from methyl tert-butyl ether and n-hexane to obtain 21.7g of (3S,6R)-3-dimethyltert-butylsilyloxy-6-[2-(2-thiophene)acetamido]-6-(4,4,5,5-tetramethyl-1,3, 2-dioxaborolan-2-yl)hexanoic acid tert-butyl ester (VII), which is a white solid having an LC purity of 99.3% and a yield of 76.4%.

### Example 9: Preparation of (3S,6R)-3-dimethyltert-butylsilyloxy-6-[2-(2-thiophene) acetamido]-6-(4,4,5,5-tetramethyl-1,3,2-dioxaborolan-2-yl)hexanoic acid tert-butyl ester (VII)

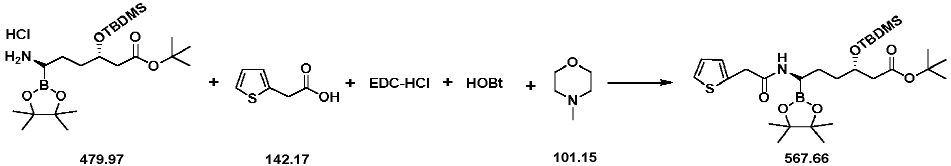

Under the protection of nitrogen, add 100g of dichloromethane, 8.5g (0.06mol) of thiopheneacetic acid, 14.8g (0.075mol) of EDC-HCl (EDCI), and 8.1g (0.06mol) of 1-hydroxybenzotriazole (HOBT) into a 250ml reaction flask equipped with a stirrer and a thermometer, and then stir after cool to 0°C. Dissolve 24.0g (0.05mol) of (3 S, 6R)-3 -dimethyl-tert-butylsilyloxy-6-amino-6-(4,4, 5,5-tetramethyl-1,3,2-dioxaborolan-2-yl )hexanoic acid tert-butyl ester hydrochloride (VI) as prepared in Example 7 into 60g of dichloromethane, and then introduce to the system together with (0.11mol) of N-methylmorpholine. Heat to the room temperature, and stir till the reaction is confirmed as completed by using the HPLC. Add 80g of water, and transfer to a separating funnel for static separation. Wash the organic phases with water, and dry out with anhydrous sodium sulfate. Reduce the pressure, distill and concentrate. Recrystallize from methyl tert-butyl ether and n-hexane to obtain 23.3g of (3S,6R)-3-dimethyltert-butylsilyloxy-6-[2-(2-thiophene)acetamido]-6-(4,4,5,5-tetramethyl-1,3, 2-dioxaborolan-2-yl)hexanoic acid tert-butyl ester (VII), which is a white solid having an LC purity of 99.4% and a yield of 82.1%.

### Example 10: Preparation of Vaborbactam (I)

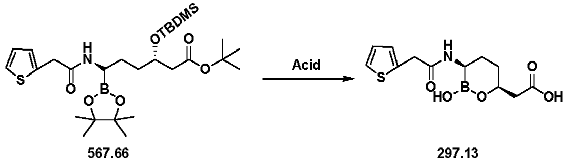

Add 80g of 1,4-dioxane and 28.4g (0.05mol) of (3 S, 6R)-3 -dimethyltert-butylsilyloxy-6-[2-(2-thiophene)acetamido]-6-(4,4,5,5-tetramethyl-1,3, 2-dioxaborolan-2-yl)hexanoic acid tert-butyl ester (VII) as obtained in Example 9 into a 250ml reaction flask equipped with a stirrer and a thermometer. After stir, add 100ml of 3mol/L dilute hydrochloric acid, heat the mixture to 90 ∼ 95°C, reflux and stir for 2 hours. Cool to the room temperature, add 100g of water and 150g of methyl tert-butyl ether, and transfer to a separating funnel for static separation. Distill and concentrate the aqueous phase under a reduced pressure. Add acetonitrile into the residues, then reduce the pressure and distill via azeotropic process for 3 times, with 80g of acetonitrile used for each time. Dissolve the residues into a 20% 1,4-dioxane/water solution, and lyophilize to form 14.3g of white powder. Add 200g of ethyl acetate and 60g of water to the white powder, stir at the room temperature for 1 hour and then wait for the formation of white sediment. Process by suction filtration, and wash the residual cake twice with ethyl acetate, 15g each time. Finally vacuum-dry to obtain 10.5g of product (I), which is a white solid having an LC purity of 99.6% and a yield of 71.0%.

The NMR data of the product are presented as follows:
¹H NMR (CD₃OD) ppm: δ 7.35 (dd, 1H), 7.05 (dd, 1H), 7.0 (dd, 1H), 4.15-4.05 (m, 1H), 3.98 (s, 2H), 2.61 (br d,1H), 2.37 (dd, 1H), 2.24 (dd, 1H), 1.74 (br d, 1H), 1.66-1.52 (m, 2H), 1.03 (br q, 1H).

### Comparative Example 1: Preparation of (3S,6R)-3-dimethyl-tert-butylsilyloxy-6-(N-(R-1-tert-butylsulfinyl)amino)-6-(4,4,5,5-tetramethyl-1,3,2-dioxaborolan-2-yl)hexanoi c acid tert-butyl ester (V₂)

Add 10g of toluene, 2g of water, 0.02g (0.12mmol) of copper sulfate, and 0.037g (0.12mmol) of triphenylphosphine oxide into a 250ml reaction flask equipped with a stirrer and a thermometer, and stir the mixture. Add 4.2g (10mmol) of S-3-dimethyl-tert-butylsilyloxy-6-(N-(R-1-tert-butylsulfinyl)imino)hexanoic acid tert-butyl ester (IV₂) as prepared in Example 4 and 3.1g (12mmol) of bis(pinacolato)diboron, and stir at the room temperature. Track and monitor the reaction by using the HPLC for 2 days, and find that only a little, less than 5%, of the raw material IV₂ is converted. Heat to 100 °C to resume the reaction, then track and monitor by using the HPLC for another 2 days, and find that only a little portion, less than 10%, of the raw material IV₂ is converted.

Comparative Example 1 shows that, in the reaction of imine addition, the presence of benzylamine is very important, the lack of benzylamine will obviously affect the reaction time and temperature, and finally almost no target product can be obtained.

### Comparative Example 2: (3S,6R)-3-dimethyl-tert-butylsilyloxy-6-(N-(R-1-tert-buty lsulfinyl)amino)-6-(4,4,5,5-tetramethyl-1,3,2-dioxaborolan-2-yl)hexanoic acid tert-butyl ester (V₂)

Add 10g of toluene, 2g of water, 0.037g (0.12mmol) of triphenylphosphine oxide and 0.054g (0.5mmol) of benzylamine into a 250ml reaction flask equipped with a stirrer and a thermometer, and then stir the mixture. Add 4.2g (10mmol) of S-3-dimethyl-tert-butylsilyloxy-6-(N-(R-1-tert-butylsulfinyl)imino)hexanoic acid tert-butyl ester (IV₂) as prepared in Example 4 and 3.1g (12mmol) of bis(pinacolato)diboron, and stir at the room temperature. Track and monitor the reaction by using the HPLC for 2 days, and find that no target product is formed, and the raw material IV₂ remains unchanged.

Comparative Example 2 shows that in the reaction of imine addition, a catalyst is indispensable, and the reaction does not occur in absence of a catalyst.

## Claims

1. A method for preparing Vaborbactam comprises the steps as follows:
(1) Reacting a compound of Formula II with a hydroxyl protecting group reagent to form a compound of Formula III; 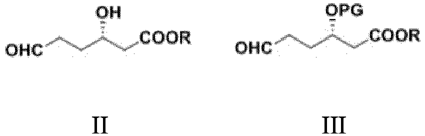 In the Structural Formulas II and III, R is methyl, ethyl, isopropyl, n-propyl, n-butyl, isobutyl, sec-butyl or tert-butyl; in the Structural Formula III, the protecting group (PG) is trimethylsilyl (TMS), tert-butyldimethylsilyl (TBDMS), benzyl (Bn), methanesulfonyl (Ms), p-toluenesulfonyl (Ts), trifluoroacetyl (TFA) or acetyl (Ac);
(2) Reacting a compound of Formula III with an amine compound by imidization to form a compound of Formula IV; 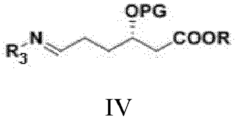 In the Structural Formula IV, R3 is hydroxyl, benzoyl, phenylacetyl, 2-thiopheneacetyl or alkylsulfinyl, and the meanings of R and PG are the same as those in Formula III;
(3) Reacting a compound of Formula IV with a borane or borate compound to form a compound of Formula V; 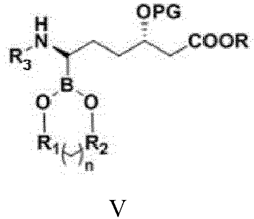 In the Structural Formula V, n is 0, 1, 2 or 3; when n is 0, R₁ and R₂ are alkyl or aryl; when n is 1, 2 or 3, R₁ and R₂ are alkyl having 1 to 4 carbon atoms; R₁ and R₂ are the same or different; and the meanings of R, PG, and R₃ are the same in Formula IV;
(4) Deprotecting a compound of Formula V to remove the protecting amino to form a compound of Formula VI; 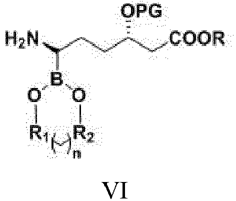 In the Structural Formula VI, the meanings of R₁, R₂, n, R and PG are the same as those in Formula V;
(5) Amidating a compound of Formula VI in the presence of an amidation reagent to form a compound of Formula VII; 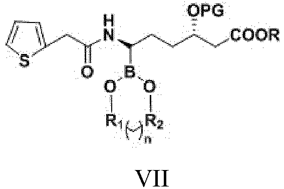 In the Structural Formula VII, the meanings of R₁, R₂, n, R and PG are the same as those in Formula V;
(6) Cyclizing and hydrolyzing a compound of Formula VII to form Vaborbactam (I).

2. A method for preparing Vaborbactam as set forth in Claim 1 is **characterized in that** it comprises one or several conditions as listed below:
a. In Step (1), reacting the compound of Formula II with a hydroxyl protecting group reagent in a Solvent A and in the presence of a base;
b. In Step (2), imidizing the compound of Formula III by using an amine compound in a Solvent B under the action of a Catalyst C;
c. In Step (3), reacting the compound of Formula IV with a borane or borate compound in a Solvent D in the presence of a Catalyst E and a ligand;
d. In Step (4), deprotecting the compound of Formula V in a Solvent F and in the presence of a deprotection reagent;
e. In Step (5), amidating the compound of Formula VI with the amidation reagent in a Solvent G under the action of the Base H; and
f. In Step (6), cyclizing and hydrolyzing the compound of Formula VII in a Solvent J and in the presence of an acid.

3. A method for preparing Vaborbactam as set forth in Claim 2 is **characterized in that** it comprises one or several conditions as listed below for carrying out Step (1) according to Item "a" therein:
i. The aforesaid Solvent A is a non-alcoholic solvent, preferably ethyl acetate, butyl acetate, acetone, methyl isobutyl ketone, acetonitrile, tetrahydrofuran, 2-methyltetrahydrofuran, 2,4-dioxane, methoxycyclopentane, methyl tert-butyl ether, or halogenated hydrocarbon solvents or benzene solvents, or any combination thereof; and the mass ratio of Solvent A to the compound of Formula II is (4 ∼ 20):1;
ii. The aforesaid base is an organic base or inorganic base, and preferably, the organic base is trimethylamine, triethylamine, tri-n-butylamine, diisopropylethylamine or imidazole, while the inorganic base is potassium carbonate, sodium carbonate or calcium carbonate, or any combination thereof; and the molar ratio of the base to the compound of Formula II is (1.0 ∼ 2.0):1;
iii. The aforesaid hydroxyl protecting group reagent is trimethylchlorosilane, trimethyliodosilane, tert-butyldimethylchlorosilane, tert-butyldimethyliodosilane, methanesulfonyl chloride, p-toluenesulfonyl chloride , benzyl chloride, benzyl bromide, trifluoroacetic acid or acetic anhydride; and the molar ratio of the hydroxyl protecting group reagent to the compound of Formula II is (1.0 ∼ 2.0):1; and
iv. The aforesaid reaction temperature of the compound of Formula II and the hydroxyl protecting group reagent is 0-60°C, preferably, 20 ∼ 40°C.

4. A method for preparing Vaborbactam as set forth in Claim 2 is **characterized in that** it comprises one or several conditions as listed below for carrying out Step (2) according to Item "b" therein:
i. The aforesaid Solvent B is methanol, ethanol, isopropanol, acetonitrile, tetrahydrofuran, 2-methyltetrahydrofuran, 2,4-dioxane, methoxycyclopentane, methyl tert-butyl ether, or halogenated hydrocarbon solvents or benzene solvents, or any combination thereof; and the mass ratio of Solvent B to the compound of Formula III is (1 ∼ 20): 1;
ii. The aforesaid Catalyst C is acetic acid, methanesulfonic acid, benzenesulfonic acid, p-toluenesulfonic acid, pyridine p-toluenesulfonic acid, copper sulfate, copper acetate, copper chloride, cuprous chloride or ferric chloride, or any combination thereof; and the molar ratio of Catalyst C to the compound of Formula III is (1 ∼ 3):1;
iii. The aforesaid amine compound is hydroxylamine, benzamide, phenylacetamide, thiopheneacetamide or alkylsulfenamide; and the molar ratio of the amine compound to the compound of Formula III is (1.0 ∼ 2.0):1; and
iv. The aforesaid imidization reaction is carried out at a temperature of -10 ∼ 100°C, preferably 0 ∼ 70°C, and more preferably 30 ∼ 50°C.

5. A method for preparing Vaborbactam as set forth in Claim 2 is **characterized in that** it comprises one or several conditions as listed below for carrying out Step (3) according to Item "c" therein:
i. The aforesaid Solvent D is methanol, ethanol, isopropanol, acetonitrile, tetrahydrofuran, 2-methyltetrahydrofuran, 1,3-dioxane, methoxycyclopentane, methyl tert-butyl ether, water, or halogenated hydrocarbon solvents or benzene solvents, or any combination thereof; and the mass ratio of Solvent D to the compound of Formula IV is (2 ∼ 20):1;
ii. The aforesaid Catalyst E is copper sulfate, copper chloride, cuprous chloride, palladium chloride, palladium acetate, tris(triphenylphosphine)rhodium(I) chloride, Grubb's catalyst, iridium/alumina or (1, 5-cyclooctadiene)(pyrimidine)(tricyclohexylphosphine)iridium(I) hexafluorophosphate; and the molar quantity of Catalyst E is 1.0 ∼ 10.0% of that of the compound of Formula IV;
iii. The aforesaid ligand is nitrogen ligand or phosphorus ligand, or preferably, a combination thereof; preferably, the nitrogen ligand is substituted imidazole or benzylamine, and the phosphorus ligand is triphenylphosphine or triphenylphosphine oxide; and the molar quantity of ligand is 1.0 ∼ 10.0% of that of the compound of Formula IV;
iv. The aforesaid borane is dialkylborane or pinacolborane; the aforesaid borate compound is trialkyl borate or biborate; the molar ratio of the borane or borate compound to the compound of Formula IV compound is 1.0 ∼ 2.0:1; and
v. The aforesaid reaction of the compound of Formula IV with the borane or borate compound is carried out at a temperature of 20 ∼ 120°C, more preferably 20 ∼ 40°C.

6. A method for preparing Vaborbactam as set forth in Claim 2 is **characterized in that** it comprises one or several conditions as listed below for carrying out Step (4) according to Item "d" therein:
i. The aforesaid Solvent F is methanol, ethanol, isopropanol, acetonitrile, tetrahydrofuran, 2-methyltetrahydrofuran, 2,4-dioxane, methoxycyclopentane, methyl tert-butyl ether, water, or halogenated hydrocarbon solvents or benzene solvents, or any combination thereof; and the mass ratio of the Solvent F to the compound of Formula V is 1 ∼ 20:1;
ii. The aforesaid deprotection reagent is an acid or a base; the acid is hydrogen chloride, sulfuric acid or phosphoric acid; the hydrogen ion concentration in the acid is 3 ∼ 8mol/L; the base is sodium hydroxide, potassium hydroxide, barium hydroxide, sodium carbonate or potassium carbonate; the molar ratio of the deprotection reagent to the compound of Formula V is (3 ∼ 7):1; and
iii. The aforesaid deprotection is carried out at a temperature of -10 ∼ 100°C, preferably -5 ∼ 30°C.

7. A method for preparing Vaborbactam as set forth in Claim 2 is **characterized in that** it comprises one or several conditions as listed below for carrying out Step (5) according to Item "e" therein:
i. The aforesaid Solvent G is methanol, ethanol, isopropanol, acetonitrile, tetrahydrofuran, 2-methyltetrahydrofuran, 2,4-dioxane, 1,4-dioxane, methoxycyclopentane, methyl tert-butyl ether, or halogenated hydrocarbon solvents or benzene solvents, or any combination thereof; and the mass ratio of the Solvent G to the compound of Formula VI is (4 ∼ 20): 1;
ii. The aforesaid amidation reagent is 2-thiopheneacetyl chloride or 2-thiopheneacetic acid; and the molar ratio of the amidation reagent to the compound of Formula VI is 1 ∼ 2.0:1;
Preferably, when the amidation reagent is 2-thiopheneacetic acid, a dehydrating and condensing agent is required; the dehydrating and condensing agent is dicyclohexylcarbodiimide (DCC), 1-ethyl-3-(3-dimethylaminopropyl)carbodiimide hydrochloride (EDCI) or 1-hydroxybenzotriazole (HOBT), or any combination thereof; and the molar ratio of the dehydrating and condensing agent to the compound of Formula VI is 1.0 ∼ 3.0:1;
iii. The aforesaid Base H is an organic base or inorganic base; the organic base is trimethylamine, triethylamine, tri-n-butylamine, diisopropylethylamine, imidazole, morpholine or N-methylmorpholine; the inorganic base is potassium carbonate, sodium carbonate or calcium carbonate, or any combination thereof; the molar ratio of the Base H to the compound of Formula VI is (1.0 ∼3.0):1; and
iv. The aforesaid amidation is carried out at a temperature of 0 ∼ 120°C, more preferably 15 ∼ 80°C.

8. A method for preparing Vaborbactam as set forth in Claim 2 is **characterized in that** it comprises one or several conditions as listed below for carrying out Step (6) according to Item "f" therein:
i. The aforesaid Solvent J is methanol, ethanol, isopropanol, acetonitrile, tetrahydrofuran, 2-methyltetrahydrofuran, 1,4-dioxane, methoxycyclopentane, methyl tert-butyl ether, or halogenated hydrocarbon solvents or benzene solvents, or any combination thereof; and the mass ratio of the Solvent J to the compound of Formula VII is (4 ∼ 20):1;
ii. The aforesaid acid is hydrochloric acid, sulfuric acid, boric acid or trifluoroacetic acid, or any combination thereof; and the molar ratio of the acid to the compound of Formula VII is (5 ∼ 7):1; and
iii. The aforesaid cyclization and hydrolysis are carried out at a temperature of 10 ∼ 100°C; and the time of cyclization and hydrolysis is 1 ∼ 10 hours.
